(19) Europäisches Patentamt
European Patent Office
Office européen des brevets

(11) EP 0 773 932 B1

(12) # FASCICULE DE BREVET EUROPEEN

(45) Date de publication et mention de la délivrance du brevet:
**26.09.2001 Bulletin 2001/39**

(21) Numéro de dépôt: **96920901.4**

(22) Date de dépôt: **05.06.1996**

(51) Int Cl.⁷: **C07D 215/26**, A61K 31/47, C07D 401/12

(86) Numéro de dépôt international:
**PCT/FR96/00845**

(87) Numéro de publication internationale:
**WO 96/40639 (19.12.1996 Gazette 1996/55)**

(54) **DERIVES DE BENZENESULFONAMIDE COMME ANTAGONISTES DE LA BRADYKININE**
BENZENSULFONAMIDDERIVATE ALS BRADYKININ ANTAGONISTEN
BENZENESULFONAMIDE DERIVATIVES USED AS BRADYKININE ANTAGONISTS

(84) Etats contractants désignés:
**AT BE CH DE DK ES FI FR GB GR IE IT LI LU MC NL PT SE**

(30) Priorité: **07.06.1995 FR 9506703**

(43) Date de publication de la demande:
**21.05.1997 Bulletin 1997/21**

(73) Titulaire: **FOURNIER INDUSTRIE ET SANTE**
**F-75008 Paris (FR)**

(72) Inventeurs:
- **DODEY, Pierre**
  **F-21121 Fontaine-lès-Dijon (FR)**
- **PRUNEAU, Didier**
  **F-21370 Pasques (FR)**
- **PAQUET, Jean-Luc**
  **F-21000 Dijon (FR)**
- **BONDOUX, Michel**
  **F-21121 Fontaine-lès-Dijon (FR)**
- **HOUZIAUX, Patrick**
  **F-78580 Bazemont (FR)**
- **BARTH, Martine**
  **F-78490 Montfort-l'Amaury (FR)**
- **OU, Khan**
  **F-21121 Hauteville-lès-Dijon (FR)**

(74) Mandataire: **Hubert, Philippe et al**
**Cabinet Beau de Loménie**
**158, rue de l'Université**
**75340 Paris Cédex 07 (FR)**

(56) Documents cités:
**EP-A- 0 261 539**
**EP-A- 0 596 406**
**EP-A- 0 622 361**
**US-A- 5 212 182**

Remarques:
Le dossier contient des informations techniques présentées postérieurement au dépôt de la demande et ne figurant pas dans le présent fascicule.

EP 0 773 932 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne de nouveaux composés de benzènesulfonamide, leur procédé de préparation et leur utilisation en thérapeutique.

Ces nouveaux composés présentent notamment une action antagoniste vis-à-vis de la bradykinine et sont utiles en thérapeutique, particulièrement pour le traitement de la douleur et de l'inflammation, et notamment dans le traitement de l'asthme et des rhinites allergiques.

## Art antérieur

On sait que l'une des possibilités de traitement de certaines pathologies à caractère douloureux et/ou inflammatoire (telles que l'asthme, la rhinite, le choc septique, la douleur dentaire, etc...) est d'antagoniser l'action de certaines hormones telles que la bradykinine ou la kallidine. En effet ces hormones peptidiques sont impliquées dans un grand nombre de processus physiologiques dont certains sont liés de façon étroite à ces pathologies.

Bien qu'actuellement aucun produit possédant ce mode d'action ne soit encore commercialisé, de nombreuses études ont été entreprises pour créer des composés susceptibles d'être antagonistes de récepteurs de la bradykinine. La bradykinine est une hormone peptidique constituée de 9 aminoacides (Arg-Pro-Pro-Gly-Phe-Ser-Pro-Phe-Arg) et la kallidine est une hormone peptidique (Lys-Arg-Pro-Pro-Gly-Phe-Ser-Pro-Phe-Arg) qui comporte un aminoacide supplémentaire (Lys) par rapport à la bradykinine. On sait que des études antérieures ont permis d'obtenir des peptides qui réagissent avec les récepteurs de la bradykinine : certains comme le Bradycor (CP.0127 de la société Cortech), l'Icatibant (HOE 140 de la société Hoechst) ["Bradycor" et "Icatibant" sont des dénominations communes internationales (DCI)] ou encore le NPC 17761 (de la société Scios-Nova) présentent une action inhibitrice de la fixation de la bradykinine sur le récepteur B2 de la bradykinine. Plus récemment, des composés non peptidiques ont été proposés comme antagonistes de la bradykinine vis-à-vis de la fixation sur le récepteur $B_2$ de la bradykinine, notamment dans US-A-5 212 182, EP-A-0596406 et EP-A-0622361. On sait en outre que certains composés de structure apparentée à ceux décrits dans les deux demandes de brevet précitées ont déjà été décrits, notamment dans DE-A-3617183 et dans EP-A-0261539, eu égard à leurs éventuelles propriétés antithrombotiques.

## But de l'invention

Il existe un besoin d'atténuer ou de supprimer chez le mammifère et surtout chez l'homme les douleurs et les inflammations.

Pour satisfaire ce besoin, on a recherché une nouvelle solution technique qui soit efficace dans le traitement des algies quelle que soit leur origine, notamment celles liées à des phénomènes inflammatoires, d'une part, et dans le traitement des inflammations, d'autre part.

Selon l'invention, on se propose de fournir une nouvelle solution technique, qui met en oeuvre une fixation compétitive au niveau du récepteur $B_2$ de la bradykinine entre (i) la bradykinine et les hormones apparentées ou analogues, et (ii) une substance antagoniste, et qui fait appel à des composés de benzènesulfonamide structurellement différents des produits connus précités et qui limitent ou inhibent substantiellement la fixation de la bradykinine et des hormones analogues sur ledit récepteur $B_2$ de la bradykinine.

Conformément à cette nouvelle solution technique on se propose de fournir, selon un premier aspect de l'invention, des composés de benzènesulfonamide en tant que produits industriels nouveaux ; selon un second aspect de l'invention, un procédé de préparation de ces composés ; et selon un troisième aspect de l'invention, l'utilisation de ces composés notamment en thérapeutique en tant qu'ingrédients actifs antalgiques et/ou anti-inflammatoires.

## Objet de l'invention

Selon la nouvelle solution technique de l'invention, on préconise en tant que produit industriel nouveau, un composé de benzènesulfonamide qui est caractérisé en ce qu'il est choisi parmi l'ensemble constitué par :

(i) les composés de formule :

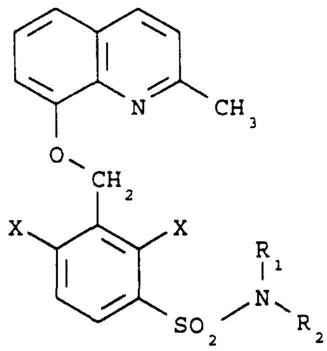

(I)

dans laquelle :

X représente un atome d'halogène,
$R_1$ et $R_2$, identiques ou différents, représentent chacun un atome d'hydrogène ou un groupe $-A-B-R_3$,
A représente une chaîne alkylène en $C_1$-$C_{12}$, linéaire ou ramifiée,
B représente

une simple liaison,
un groupe phénylène de structure

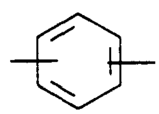

par rapport auquel les substituants A et $R_3$ sont en position ortho, méta ou para, ou
un groupe indolyle divalent de structure

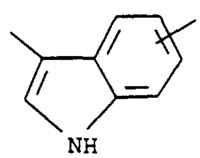

$R_3$ représente -H, -OH, $-NR_4R_5$ ou $-COR_6$,
$R_6$ représente un groupe -OH, $-OCH_3$, $-OC_2H_5$ ou $-NR_4R_5$,
$R_4$ et $R_5$, identiques ou différents représentent chacun un atome d'hydrogène, un groupe alkyle en $C_1$-$C_4$ à chaîne hydrocarbonée linéaire ou ramifiée, un groupe $-(CH_2)_n-OH$, un groupe $-(CH_2)_n-N(CH_3)_2$ ou un groupe $-CO-CH_3$,
n représente un nombre entier ayant pour valeur 2, 3 ou 4 ; et,

(ii) leurs sels d'addition.

Les benzènesulfonamides selon l'invention peuvent être préparés suivant une méthode connue en soi par application de mécanismes réactionnels classiques. Néanmoins, le procédé que l'on préconise selon l'invention pour la préparation des composés de benzènesulfonamide de formule I et de leurs sels d'addition est caractérisé en ce qu'il comprend la réaction de condensation de la 8-hydroxy-2-méthylquinoléine sous la forme phénate de formule :

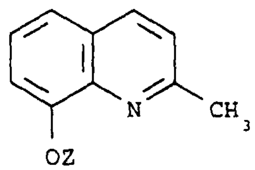

(VIII)

où Z est un atome de métal alcalin, avec un toluènesulfonamide halogéné de formule :

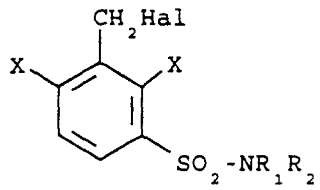

(IX)

dans laquelle Hal représente un atome d'halogène, et X, $R_1$ et $R_2$ sont définis comme indiqué ci-dessus, les fonctions (notamment alcool -OH, amine primaire $-NH_2$ et amine secondaire -NH-) susceptibles d'être contenues dans $R_1$ et/ou $R_2$ et d'intervenir dans la réaction VIII + IX → I étant protégées.

On préconise également l'utilisation d'une substance choisie parmi les composés de formule I et leurs sels d'addition non-toxiques pour l'obtention d'un médicament destiné à une utilisation en thérapeutique vis-à-vis des pathologies impliquant la bradykinine ou ses analogues, en particulier vis-à-vis des algies, et notamment dans le traitement ou la prévention de pathologies liées à des états inflammatoires ou douloureux.

**Description détaillée de l'invention**

Dans la formule générale I des composés de l'invention, l'atome d'halogène représente l'atome de fluor, de chlore, de brome ou d'iode, l'halogène préféré étant l'atome de chlore.

Par groupe alkyle en $C_1$-$C_4$, on entend les groupes méthyle, éthyle, propyle, 1-méthyléthyle, butyle, 1,1-diméthyléthyle, 1-méthylpropyle et 2-méthylpropyle.

Par "sels d'addition", on entend les sels d'addition d'acide obtenus par réaction d'un composé de formule I avec un acide minéral ou un acide organique. Les acides minéraux préférés pour salifier un composé basique de formule I sont les acides chlorhydrique, bromhydrique, phosphorique et sulfurique. Les acides organiques préférés pour salifier un composé basique de formule I sont les acides méthanesulfonique, maléique, fumarique, oxalique, citrique et trifluoroacétique.

Par commodité, les composés de benzènesulfonamide, qui interviennent en tant qu'agents antagonistes, sont indifféremment mentionnés comme étant des antagonistes de la bradykinine ou des antagonistes du récepteur $B_2$ de la bradykinine.

Parmi les composés de formule I selon l'invention, on préfère du point de vue de l'activité pharmacologique ceux appartenant à l'ensemble constitué par les composés de formule :

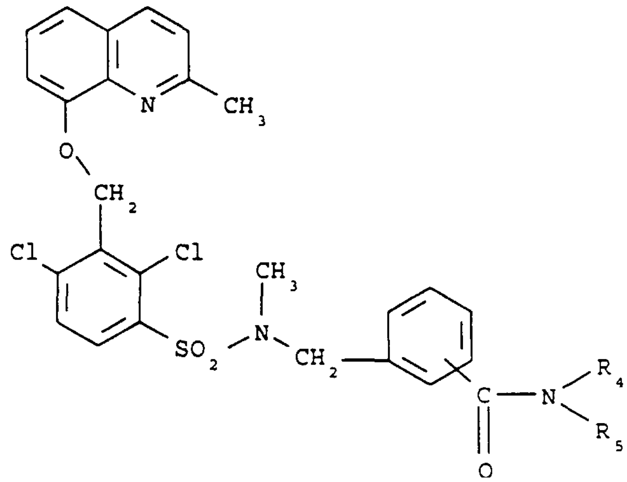

dans laquelle le groupe amide peut être en position ortho, méta ou para et, $R_4$ et $R_5$, identiques ou différents, représentent chacun :

- un atome d'hydrogène,
- un groupe alkyle en $C_1$-$C_4$, linéaire ou ramifié,
- un groupe $-(CH_2)_n-OH$, ou
- un groupe $-(CH_2)_n-N(CH_3)_2$,
- où n représente un nombre entier valant 2, 3 ou 4 ; et,

leurs sels d'addition d'acide non-toxiques.

Le procédé général pour la préparation des composés de benzènesulfonamide selon la réaction (1)

$$(1) \qquad VIII + IX \rightarrow I$$

est mis en oeuvre dans un solvant aprotique [tel que notamment le diméthylformamide (DMF) ou le tétrahydrofuranne (THF)], à une température comprise entre 0 et 50°C, le métal alcalin Z étant Li, K ou mieux Na.

Le phénate VIII peut être préparé *in situ* pour réaliser la réaction VIII + IX → I. Dans cette réaction, il peut être intéressant que l'atome d'halogène Hal du composé IX ait une électronégativité inférieure ou égale à celle de l'atome d'halogène X ; d'un point de vue pratique : si X est Cl, alors Hal sera avantageusement Br.

Enfin, la réaction VIII + IX → I implique la protection des fonctions réactives susceptibles d'être présentes dans les groupes $R_1$ et/ou $R_2$. Ces fonctions réactives comprennent les groupements -OH, $-NH_2$ et -NH- qui comportent un atome d'hydrogène réactif pouvant perturber le déroulement de la réaction (1) souhaitée.

A titre d'illustration, des modalités de mise en oeuvre de la réaction VIII + IX → I sont données dans la préparation III ci-après.

Pour la préparation de l'ensemble des composés de formule I, on procède selon le schéma réactionnel suivant :

(α) on synthétise, selon la réaction (1) précitée, un composé de formule :

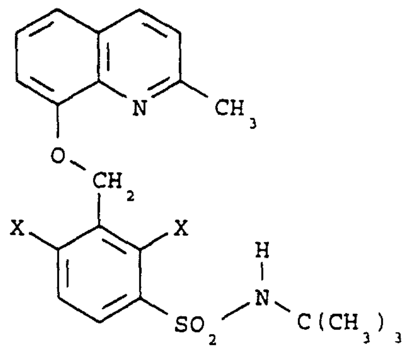

(V)

[qui est un composé de formule I où $R_2$ est H et $R_1$ est $C(CH_3)_3$],

(β) si nécessaire, on traite le composé de formule V pour remplacer le groupe $R_2$ = H par un groupe $R_2$ différent de H et obtenir un composé de formule :

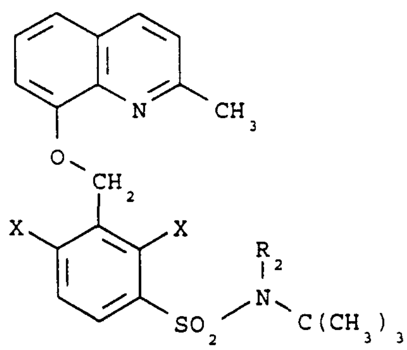

(Va)

(γ) si nécessaire, on traite le composé de formule Va, ainsi obtenu, pour remplacer le groupe $R_1 = C(CH_3)_3$ par l'atome d'hydrogène et obtenir un composé de formule :

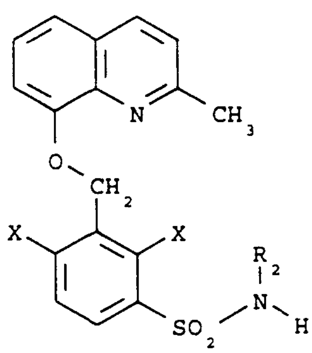

(Vb)

puis,

(δ) si nécessaire, on traite le composé de formule Vb, ainsi obtenu, pour remplacer le groupe $R_1$ = H par un groupe $R_1$ différent de H et obtenir un composé de formule I où $R_1$ et $R_2$ sont chacun différents de H,

et,

(ε) si nécessaire, on obtient le sel d'addition d'acide de la base libre résultant du stade (β), (γ) ou (δ) [en faisant réagir ladite base libre avec l'acide choisi pour la salification].

Les stades (β), (γ) et (δ) impliquent la protection puis la déprotection de tout groupe réactif pouvant être contenu dans $R_1$ et/ou $R_2$.

D'un point de vue pratique, le schéma réactionnel précité mettra en oeuvre l'une des variantes choisies parmi l'ensemble constitué par la variante A qui comprend les étapes consistant à :

1) faire réagir un composé de formule :

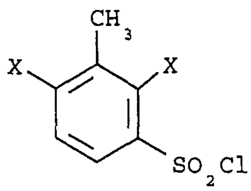

(II)

dans laquelle X représente un atome d'halogène, avec de la tert-butylamine (autre nomenclature : 1,1-diméthyléthanamine) en excès, dans un solvant comme par exemple du dichlorométhane, à température ambiante pendant 1 à 3 heures pour obtenir un composé de formule :

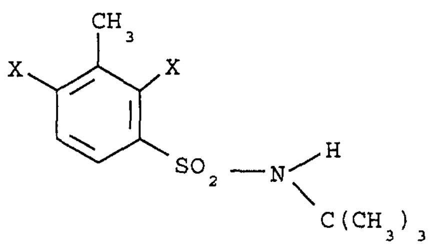

(III)

dans laquelle X représente un atome d'halogène,

2) halogéner le composé III obtenu ci-dessus à l'aide de N-halogénosuccinimide (de préférence le N-bromosuccinimide), dans un solvant halogéné notamment le tétrachlorure de carbone et de préférence en présence d'un initiateur radicalaire, notamment l'AIBN [autre nomenclature : 2,2'-azobis(2-méthylpropionitrile) ou 2,2'-azobis(isobutyronitrile)] et/ou d'un rayonnement ultra violet, pendant 2 à 24 heures, à une température comprise entre 30°C et 100°C, pour obtenir un composé de formule :

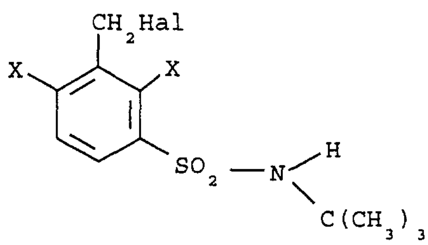

(IV)

dans laquelle X et Hal représentent chacun un atome d'halogène,

3) condenser 1e composé IV, ainsi obtenu, avec la 8-hydroxy-2-méthylquinoléine en faisant réagir préalablement sur cette dernière de l'hydrure de sodium, en présence d'un solvant (notamment le DMF), à une température comprise entre 0°C et 50°C, pour obtenir le composé de formule :

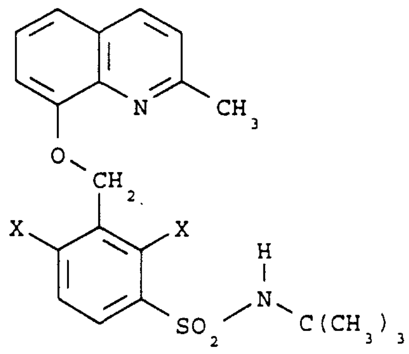

(V)

dans laquelle X représente un atome d'halogène,

4) faire réagir le composé de formule V ainsi obtenu avec de l'hydrure de sodium, dans un solvant organique anhydre (notamment le DMF), à une température comprise entre 0°C et 30°C, puis faire réagir sur le dérivé sodé ainsi obtenu un composé de formule Y-$R_2$ dans laquelle :

Y est un atome d'halogène, (notamment un atome d'iode, un atome de brome ou un atome de chlore), un groupe méthanesulfonyle ou un groupe toluènesulfonyle, et
$R_2$ représente un groupe alkyle en $C_1$-$C_{12}$ ou un groupe arylalkyle, ces deux groupes étant éventuellement substitués par un ou plusieurs groupements fonctionnels protégés pour ne pas réagir avec le sulfonamide sodé,

pour obtenir un composé de formule

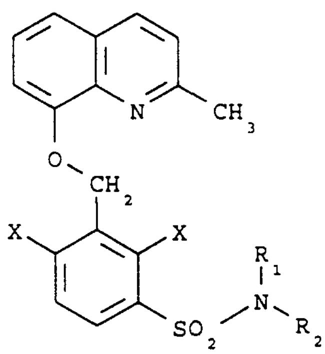

(I)

dans laquelle :

X représente un atome d'halogène,
$R_1$ représente le groupe 1,1-diméthyléthyle, et
$R_2$ conserve la même signification que ci-dessus ;

la variante B qui comprend les étapes consistant à :

1) traiter un composé de formule I, obtenu à l'étape 4) de la variante A, dans laquelle $R_2$ représente le groupe méthyle ou le groupe phénylméthyle, et $R_1$ représente le groupe 1,1-diméthyléthyle, à l'aide d'un excès d'acide (notamment l'acide trifluoroacétique), en présence d'un capteur de cations (notamment l'anisole), à une température comprise entre 20°C et 60°C, pendant 4 à 24 heures, pour obtenir un composé de formule :

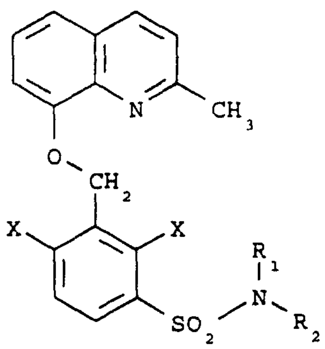

(I)

dans laquelle :

X représente un atome d'halogène,
$R_1$ représente un groupe méthyle ou un groupe phénylméthyle, et
$R_2$ représente un atome d'hydrogène,

2) traiter le composé de formule I obtenu au stade B 1) ci-dessus avec un composé de formule $YR_2$ dans les conditions identiques à celles préconisées au stade 4) de la variante A pour obtenir un composé de formule :

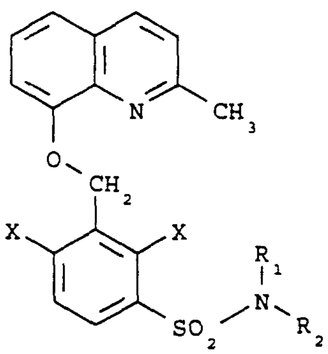

(I)

dans laquelle :

X représente un atome d'halogène,
$R_1$ représente un groupe méthyle ou un groupe phénylméthyle,
$R_2$ représente un groupe alkyle en $C_1$-$C_{12}$ ou un groupe arylalkyle, ces deux groupes étant éventuellement substitués par des groupements fonctionnels protégés pour ne pas réagir avec le groupe sulfonamide sodé,

3) si nécessaire, déprotéger le ou les groupements fonctionnels portés par le groupe $R_2$ du composé de formule I, obtenu à l'un des stades A 4) ou B 2) ci-dessus (notamment par hydrolyse d'un ester ou déprotection d'une amine), pour obtenir un composé de formule I dont le groupe $R_2$ est porteur d'un groupe fonctionnel libre, par exemple une fonction acide carboxylique ou une fonction amine primaire libres,

4) si nécessaire, faire réagir le groupe fonctionnel libéré au stade précédent pour obtenir un dérivé, par exemple un amide par réaction d'un groupe acide carboxylique avec un composé porteur d'une fonction amine primaire ou secondaire,

5) si nécessaire, obtenir un sel d'addition d'un composé de formule I initialement obtenu sous forme de base libre, en faisant réagir ladite base libre avec l'acide choisi pour la salification.

Par groupement fonctionnel protégé, on entend ici notamment :

- un groupe NH ou $NH_2$ protégé par un ou deux groupes amino-protecteurs,
- un groupe ester,
- un groupe carboxamide N-mono ou -disubstitué, ou
- un groupe OH protégé par un groupe hydroxy-protecteur.

Les groupes amino-protecteurs qui conviennent ici sont ceux qui interviennent de façon usuelle dans le domaine de la synthèse peptidique, en particulier les groupes alcoxycarbonyle, notamment le groupe Boc (t-butyloxycarbonyle). Les groupes hydroxy-protecteurs sont par exemple des groupes silyle (notamment triméthylsilyle ou diméthyl-t-butylsilyle), ou des groupes de type benzyle.

Pour illustrer l'étape 3) de la variante B, on procède comme suit :

réaction d'un composé de formule :

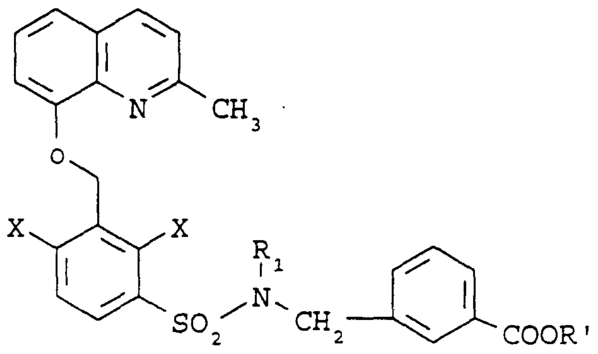

(VI)

dans laquelle :

X représente un atome d'halogène,
$R_1$ représente un groupe alkyle en $C_1$-$C_4$ ou arylalkyle, et
R' représente un groupe méthyle ou éthyle,

avec une solution d'hydroxyde de sodium, en présence d'un solvant comme par exemple du méthanol, à une température comprise entre 40°C et 80°C, pendant 0,5 à 5 heures, pour obtenir un composé de formule VI dans laquelle X et $R_1$ conservent la même signification que ci-dessus et R' représente un atome d'hydrogène.

Pour illustrer l'étape 4) de la variante B, on procède comme suit : réaction d'un composé de formule VI ainsi obtenu, où R' représente un atome d'hydrogène, avec une amine de formule $H-NR_4R_5$,
dans laquelle :

$R_4$ et $R_5$, identiques ou différents représentent chacun un atome d'hydrogène, un groupe alkyle en $C_1$-$C_4$ (linéaire ou ramifié), un groupe $(CH_2)_n$-OH, ou un groupe $(CH_2)_n-N(CH_3)_2$, où n représente un nombre entier ayant pour valeur 2, 3 ou 4,

dans un solvant comme par exemple du dichlorométhane et en présence d'agents de couplage comme par exemple le N,N'-dicyclohexylcarbodiimide et le 1-hydroxybenzotriazole, à une température proche de la température ambiante, pendant 10 à 48 heures, pour obtenir un composé de formule :

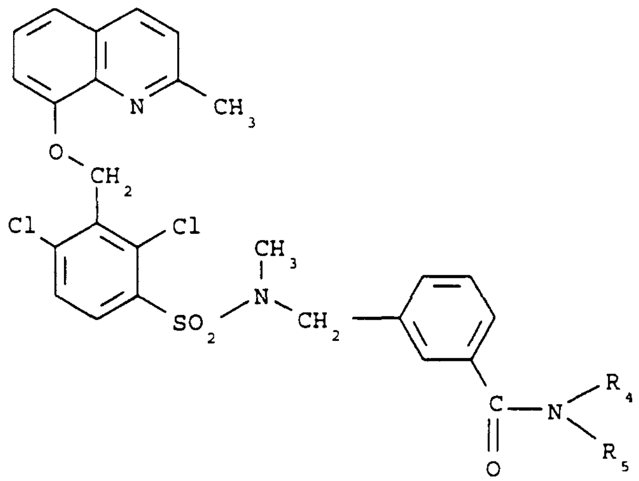

(VII)

dans laquelle X, $R_4$, $R_5$ conservent les mêmes significations que ci-dessus.

Le composé de formule IV, dans lequel X est un atome de chlore et Hal est l'atome de brome, est nouveau et constitue l'un des objets de l'invention.

L'invention sera mieux comprise à la lecture des exemples de préparation qui suivent et des résultats d'essais pharmacologiques obtenus avec certains composés selon l'invention. La nomenclature utilisée dans ces exemples est celle préconisée dans les Chemical Abstracts.

Dans la partie expérimentale, les "préparations" sont relatives aux composés intermédiaires et les "exemples" sont relatifs aux produits selon l'invention.

Certains composés sont caractérisés par les données spectrales obtenues en résonance magnétique nucléaire (RMN) ; dans ce cas, les caractéristiques spectrales sont données pour le proton ($^1H$), et on indique le déplacement chimique des protons par rapport au signal proton du tétraméthylsilane avec, entre parenthèses, la forme du signal (s pour singulet, d pour doublet, t pour triplet, q pour quadruplet, m pour multiplet, sl pour signal large) et le nombre de protons pour le signal. A titre indicatif, les spectres RMN$^1$H ont été réalisés à 250 MHz.

Les points de fusion (F) indiqués ci-après sont en général mesurés à l'aide d'un banc Koffler et ne sont pas corrigés, il s'agit alors de points de fusion instantanée.

**PREPARATION I**

**2,4-dichloro-N-(1,1-diméthyléthyl)-3-méthylbenzènesulfonamide**

On prépare une solution de 100 g (0,385 mole) de chlorure de 2,4-dichloro-3-méthylbenzènesulfonyle dans 1 litre de dichlorométhane et on ajoute doucement 112,5 g (1,54 mole) de 1,1-diméthyléthanamine, à température ambiante (20-25°C). On maintient le milieu réactionnel sous agitation pendant 2 heures après la fin de l'addition puis on hydrolyse sur de l'eau. Après extraction au dichlorométhane, les phases organiques rassemblées sont lavées à l'eau jusqu'à pH neutre puis séchées sur sulfate de sodium et concentrées sous pression réduite. Le résidu solide est recristallisé dans le méthylcyclohexane. On obtient ainsi 80 g du produit attendu sous forme de solide blanc (Rendement = 70 %).
F = 148-150°C

**PREPARATION II**

**2,4-dichloro-N-(1,1-diméthyléthyl)-3-(bromométhyl)benzènesulfonamide**

A une solution de 77 g (0,26 mole) du composé obtenu selon la préparation I, dans 1,2 litre de tétrachlorure de carbone, on ajoute 2,1 g (0,012 mole) d'AIBN et 55,6 g (0,312 mole) de N-bromosuccinimide. Le mélange réactionnel est ensuite agité sous irradiation ultra violet et porté à reflux du solvant pendant 4 heures. Après refroidissement, le mélange est hydrolysé sur de l'eau puis extrait avec du dichlorométhane. Les phases organiques rassemblées sont lavées à l'eau, séchées sur sulfate de sodium et concentrées sous pression réduite. Le résidu est recristallisé dans l'isopropanol et on obtient ainsi 91 g du produit attendu sous forme de solide cristallisé blanc (Rendement = 94 %).
F = 157°C

**PREPARATION III**

**2,4-dichloro-N-(1,1-diméthyléthyl)-3-[(2-méthylquinolin-8-yl)oxyméthyl]-benzènesulfonamide (Exemple 29)**

On prépare une solution de 39,8 g (0,25 mole) de 2-méthyl-8-hydroxy-quinoléine dans 400 ml de diméthylformamide (DMF) et on ajoute, à température ambiante, par petites portions, 7,5 g (0,25 mole) d'hydrure de sodium à 80 % dans l'huile. On agite le mélange pendant 30 min puis on refroidit à 0°C et on ajoute goutte à goutte 103,15 g (0,275 mole) de composé obtenu selon la préparation II en solution dans 120 ml de DMF. On laisse ensuite le mélange sous agitation à température ambiante pendant une heure puis on hydrolyse sur de l'eau et extrait au dichlorométhane. Les phases organiques sont lavées à l'eau, séchées sur sulfate de sodium et concentrées sous pression réduite. On obtient ainsi 111,2 g du produit attendu sous forme de solide pulvérulent beige (Rendement = 98 %).
F = 191°C

**Exemple 1**

**2,4-dichloro-N-(1,1-diméthyléthyl)-N-méthyl-3-[(2-méthylquinolin-8-yl)-oxyméthyl]benzènesulfonamide**

A une solution de 90,68 g (0,2 mole) du composé obtenu selon la préparation III dans 650 ml de DMF, on ajoute 6,3 g (0,21 mole) d'hydrure de sodium à 80 % dans l'huile, par petites portions, à température ambiante (20-25°C). Le mélange est maintenu sous agitation pendant 45 min, puis on ajoute goutte à goutte 31,22 g (0,22 mole) d'iodure de méthyle en solution dans 10 ml de DMF. Après une heure sous agitation, le milieu réactionnel est versé sur de l'eau glacée. Le précipité formé est séparé par filtration, lavé à l'eau sur le filtre et séché sous vide à 70°C. On obtient ainsi 89,9 g du produit attendu sous forme d'une poudre beige clair (Rendement = 96 %).
F = 160-162°C

**Exemple 2**

**2,4-dichloro-N-méthyl-3-[(2-méthylquinolin-8-yl)oxyméthyl]benzènesulfonamide**

On prépare un mélange de 88,8 g (0,19 mole) du composé obtenu selon l'exemple 1 dans 250 ml d'acide chlorhydrique 10N et on maintient ce mélange sous agitation pendant une heure à température ambiante. On verse ensuite le milieu réactionnel sur de l'eau glacée puis on filtre le solide en suspension. Le produit brut ainsi obtenu est repris avec une solution d'hydroxyde de sodium 2N et extrait avec du dichlorométhane. La phase organique est lavée à l'eau, séchée sur sulfate de sodium et concentrée sous pression réduite. On obtient ainsi 74,5 g du produit attendu sous forme d'un solide blanc (Rendement = 95 %).
F = 174-176°C

**Exemple 3**

**2,4-dichloro-3-[(2-méthylquinolin-8-yl)oxyméthyl]benzènesulfonamide**

On prépare un mélange de 2,26 g ($5.10^{-3}$ mole) du composé obtenu selon la préparation III et de 0,59 g ($5.10^{-3}$ mole) d'anisole, puis on ajoute 20 ml d'acide trifluoroacétique. La solution obtenue est agitée pendant 12 heures à température ambiante puis pendant 5 heures à 40°C. L'acide trifluoroacétique est ensuite chassé sous pression réduite. Le résidu obtenu est amené à neutralité à l'aide d'une solution d'hydroxyde de sodium 1N. Le produit est extrait par de l'acétate d'éthyle. La phase organique est lavée à l'eau, séchée puis concentrée sous pression réduite. Le solide obtenu est purifié par recristallisation dans le toluène et on obtient ainsi 1,4 g du produit attendu sous forme d'un solide cristallisé blanc cassé (Rendement = 70 %).
F = 206-208°C

**Exemple 4**

**2,4-dichloro-N-méthyl-N-(phénylméthyl)-3-[(2-méthylquinolin-8-yl)-oxyméthyl]benzènesulfonamide**

A une solution de 822 mg ($2.10^{-3}$ mole) du composé obtenu selon l'exemple 2, dans 5 ml de DMF, on ajoute 60 mg ($2.10^{-3}$ mole) d'hydrure de sodium en suspension dans de l'huile. Après 30 minutes d'agitation à température ambiante, on ajoute 376 mg ($2,2.10^{-3}$ mole) de bromure de benzyle et on maintient sous agitation pendant une heure à 40°C. Après refroidissement, le mélange réactionnel est versé sur de l'eau et extrait avec de l'acétate d'éthyle. Les

phases organiques sont lavées à l'eau, séchées sur sulfate de sodium et concentrées sous pression réduite. Le solide obtenu est recristallisé dans l'acétate d'éthyle et on obtient ainsi 750 mg du produit attendu sous forme d'une poudre blanche (Rendement = 75 %).
F = 154°C

**Exemple 5**

**2,4-dichloro-N-(1,1-diméthyléthyl)-N-(phénylméthyl)-3-[(2-méthylquinolin-8-yl)oxyméthyl]benzènesulfonamide**

En opérant de façon analogue au procédé de l'exemple 4, au départ du composé obtenu selon la préparation III et de bromure de benzyle, on obtient le produit attendu avec un rendement de 83 %.
F = 167-168°C

**Exemple 6**

**2,4-dichloro-N-(phénylméthyl)-3-[(2-méthylquinolin-8-yl)oxyméthyl]-benzènesulfonamide**

En opérant de façon analogue au procédé de l'exemple 3, au départ du composé obtenu selon l'exemple 5, on obtient le produit attendu avec un rendement de 77 %.
F = 140-142°C

**Exemple 7**

**2,4-dichloro-N-méthyl-N-[(indol-3-yl)méthyl]-3-[(2-méthylquinolin-8-yl)-oxyméthyl]benzènesulfonamide**

En opérant de façon analogue au procédé de l'exemple 1, au départ du composé obtenu selon l'exemple 2 et de méthosulfate de triméthylgramine (méthanesulfonate de N,N,N-triméthyl-indol-3-yl-méthanaminium), et après purification par chromatographie sur gel de silice l'éluant étant un mélange dichlorométhane/acétate d'éthyle (9/1, v/v), on obtient le produit attendu sous forme d'un solide blanc avec un rendement de 72 %.
F = 218°C

**PREPARATION IV**

**2,4-dichloro-N-méthyl-N-[(4-nitrophényl)méthyl]-3-[(2-méthyl-quinolin-8-yl)oxyméthyl]benzènesulfonamide**

En opérant de façon analogue au procédé de l'exemple 4, au départ du composé obtenu selon l'exemple 2 et de 4-nitro-$\alpha$-bromotoluène (i.e. bromure de 4-nitrobenzyle), on obtient, après recristallisation dans l'acétate d'éthyle, le produit attendu avec un rendement de 61 %.
F = 185°C

**Exemple 8**

**2,4-dichloro-N-méthyl-N-[(4-aminophényl)méthyl]-3-[(2-méthylquinolin-8-yl)oxyméthyl]benzènesulfonamide, dichlorhydrate**

On prépare une solution de 0,56 g ($1{,}03.10^{-3}$ mole) du composé obtenu selon la préparation IV dans 4 ml de méthanol et on ajoute 4 ml d'acide chlorhydrique concentré, puis 0,173 g ($3{,}1.10^{-3}$ mole) de poudre de fer. Le mélange réactionnel est chauffé à reflux sous agitation pendant 3 heures puis, après refroidissement, versé sur de l'eau glacée. Le mélange est amené à pH 8 à l'aide d'une solution d'hydroxyde de sodium 1N et le précipité formé est filtré, puis purifié par chromatographie sur gel de silice l'éluant étant un mélange dichlorométhane/acétate d'éthyle (98/2, v/v). Le solide obtenu est recristallisé dans un mélange toluène/éther isopropylique. Le composé ainsi obtenu est repris en solution dans de l'éthanol et additionné d'une solution saturée de chlorure d'hydrogène dans l'éthanol. Les cristaux formés sont filtrés et séchés sous pression réduite; on obtient ainsi 0,3 g du produit attendu sous forme de cristaux blancs légèrement hygroscopiques (Rendement = 50%).
F = 190°C

**PREPARATION V**

**2,4-dichloro-N-méthyl-N-[[4-[(1,1-diméthyléthyl)(diméthyl)silyloxy]-phényl]méthyl]-3-[(2-méthylquinolin-8-yl) oxyméthyl]benzènesulfonamide**

En opérant de façon analogue au procédé de l'exemple 4, au départ du composé obtenu selon l'exemple 2 et d'α-bromo-4-[(1,1-diméthyléthyl)(diméthyl)silyloxy]toluène, et après purification par chromatographie sur gel de silice l'éluant étant un mélange toluène/acétate d'éthyle (8/2, v/v), on obtient le produit attendu sous forme d'un solide blanc crème avec un rendement de 78 %.
F = 95°C

**Exemple 9**

**2,4-dichloro-N-méthyl-N-[(4-hydroxyphényl)méthyl]-3-[(2-méthylquinolin-8-yl)oxyméthyl]benzènesulfonamide**

On ajoute 10 ml d'une solution d'acide chlorhydrique 5N à une suspension de 1,7 g ($2,69.10^{-3}$ mole) du composé obtenu selon la préparation V dans 50 ml de méthanol, puis on agite le mélange résultant pendant 30 minutes à température ambiante. Après évaporation du méthanol sous pression réduite, le résidu obtenu est repris avec de l'eau et le mélange est neutralisé avec une solution d'ammoniaque. Le précipité formé est filtré, lavé à l'eau, séché puis recristallisé dans du méthanol. On obtient ainsi 1,18 g du produit attendu sous forme d'un solide cristallisé blanc (Rendement = 85 %).
F = 241°C

**PREPARATION VI**

**2,4-dichloro-N-méthyl-N-[[3-(méthoxycarbonyl)phényl]méthyl]-3-[(2-méthylquinolin-8-yl)oxyméthyl]benzènesulfonamide**

En opérant de façon analogue au procédé de l'exemple 4, au départ du composé obtenu selon l'exemple 2 et de 3-(bromométhyl)benzoate de méthyle, et après purification par chromatographie sur gel de silice l'éluant étant un mélange dichlorométhane/acétate d'éthyle (98/2, v/v), on obtient le produit attendu sous forme d'un solide blanc avec un rendement de 58 %.
F = 180°C

**PREPARATION VII**

**2,4-dichloro-N-méthyl-N-[(3-carboxyphényl)méthyl]-3-[(2-méthylquinolin-8-yl)oxyméthyl]benzènesulfonamide**

On ajoute 13 ml ($13.10^{-3}$ mole) d'une solution aqueuse d'hydroxyde de sodium N à une suspension de 3,6 g ($6,4.10^{-3}$ mole) du composé obtenu selon la préparation VI dans 50 ml de méthanol. On porte le mélange réactionnel à reflux pendant 4 heures puis on concentre sous pression réduite. Le résidu est repris dans de l'eau puis on acidifie jusqu'à pH 2 à l'aide d'une solution d'acide chlorhydrique 1N. Le précipité formé est filtré et séché. On obtient ainsi 2,3 g du produit attendu sous forme d'un solide blanc pulvérulent (Rendement = 66 %).
F = 204°C

**Exemple 10**

**2,4-dichloro-N-méthyl-N-[[3-(aminocarbonyl)phényl]méthyl]-3-[(2-méthylquinolin-8-yl)oxyméthyl]benzènesulfonamide**

On prépare une solution de 0,5 g ($0,92.10^{-3}$ mole) de l'acide obtenu selon la préparation VII, dans 10 ml de dichlorométhane et 2 ml de méthanol et on ajoute 0,264 g ($1,38.10^{-3}$ mole) de chlorhydrate de 1-(3-diméthylaminopropyl)-3-éthylcarbodiimide, 0,187 g ($1,38.10^{-3}$ mole) de 1-hydroxy-7-aza-benzotriazole, puis 1,5 ml d'une solution saturée d'ammoniac dans l'éthanol. Le mélange réactionnel est agité à température ambiante pendant 20 heures. On ajoute du dichlorométhane puis on lave la phase organique à l'aide d'une solution de bicarbonate de sodium puis avec de l'eau jusqu'à neutralité. La phase organique est ensuite séchée et concentrée sous pression réduite. Le résidu

solide est recristallisé dans un mélange toluène/éther isopropylique et on obtient ainsi 0,3 g du produit attendu sous forme d'un solide blanc (Rendement = 60 %)
F = 208°C

**Exemple 11**

**2,4-dichloro-N-méthyl-N-[[3-(méthylaminocarbonyl)phényl]méthyl]-3-[(2-méthylquinolin-8-yl)oxyméthyl]benzènesulfonamide**

En opérant de façon analogue au procédé de l'exemple 10, mais en utilisant une solution de méthylamine dans l'éthanol, on obtient, après purification par chromatographie sur gel de silice l'éluant étant un mélange dichlorométhane/acétate d'éthyle (7/3, v/v) puis recristallisation dans l'acétate d'éthyle, le produit attendu sous forme d'un solide blanc avec un rendement de 55 %.
F = 195°C

**Exemple 12**

**2,4-dichloro-N-méthyl-N-[[3-[(diméthylamino)carbonyl]phényl]méthyl]-3-[(2-méthylquinolin-8-yl)oxyméthyl] benzènesulfonamide.**

En opérant de façon analogue au procédé de l'exemple 10, au départ d'une solution de diméthylamine dans l'éthanol, et après purification par chromatographie sur gel de silice l'éluant étant un mélange dichlorométhane/acétate d'éthyle (75/25, v/v) puis recristallisation dans le mélange toluène/éther isopropylique, on obtient le produit attendu sous forme de poudre blanche avec un rendement de 76 %.
F = 194°C

**Exemple 13**

**2,4-dichloro-N-méthyl-N-[[3-[(3-hydroxypropyl)aminocarbonyl]-phényl]méthyl]-3-[(2-méthylquinolin-8-yl)oxyméthyl]benzènesulfonamide**

En opérant de façon analogue au procédé de l'exemple 10, au départ de 3-amino-propanol, et après purification par chromatographie sur gel de silice l'éluant étant un mélange toluène/éther isopropylique, on obtient le produit attendu sous forme d'un solide blanc pulvérulent avec un rendement de 78 %.
F = 98°C

**Exemple 14**

**2,4-dichloro-N-méthyl-N-[[3-[[2-(diméthylamino)éthyl]aminocarbonyl]-phényl]méthyl]-3-[(2-méthylquinolin-8-yl)oxyméthyl]benzènesulfonamide**

En opérant de façon analogue au procédé de l'exemple 10, au départ de N,N-diméthyléthylènediamine, après purification par chromatographie sur gel de silice l'éluant étant un mélange dichlorométhane/méthanol (9/1 ,v/v) puis recristallisation dans le mélange acétate d'éthyle/éther isopropylique, on obtient le produit attendu sous forme d'un solide blanc cristallisé avec un rendement de 60 %.
F = 142°C

**Exemple 15**

**2,4-dichloro-N-méthyl-N-(2-phényl-éthyl)-3-[(2-méthylquinolin-8-yl)-oxyméthyl]benzènesulfonamide**

En opérant de façon analogue au procédé de l'exemple 4, au départ de (2-iodoéthyl)benzène, et après purification par chromatographie sur gel de silice l'éluant étant un mélange toluène/acétate d'éthyle (95/5,v/v), on obtient le produit attendu avec un rendement de 15 %.
F = 156-160°C

**PREPARATION VIII**

**Acide (7-bromo-heptyl)(1,1-diméthyléthoxycarbonyl)carbamique, 1,1 diméthyléthyl ester**

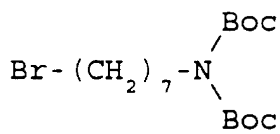

On prépare une solution de 1,15 g ($5,3.10^{-3}$ mole) d'acide iminodicarboxylique, bis(1,1-diméthyléthyl) ester dans 40 ml de DMF et on ajoute 0,175 g ($5,8.10^{-3}$ mole) d'hydrure de sodium à 80 % dans de l'huile. Après 3 heures sous agitation à température ambiante, on ajoute 6 g ($23,25.10^{-3}$ moles) de 1,7-dibromoheptane et on continue l'agitation pendant 20 heures à 60° C. Le milieu réactionnel est ensuite versé sur une solution saturée de chlorure de sodium et extrait au pentane. La phase organique est lavée à l'eau, séchée sur sulfate de sodium et concentrée sous pression réduite. Le produit brut est purifié par chromatographie sur gel de silice l'éluant étant un mélange toluène/acétate d'éthyle (9/1, v/v). On obtient ainsi 0,43 g du produit attendu sous forme d'une huile incolore (Rendement = 20,7 %).
RMN$^1$H ($CDCl_3$) : 1,50 (m,26H) ; 1,85 (q,2H) ; 3,40 (t,2H) ; 3,55 (t,2H).

**PREPARATION IX**

**2,4-dichloro-N-méthyl-N-[7-[bis(1,1-diméthyléthoxycarbonyl)amino]-heptyl]-3-[(2-méthylquinolin-8-yl)oxyméthyl]benzènesulfonamide**

En opérant de façon analogue au procédé de l'exemple 4, au départ du composé obtenu selon la préparation VIII, et après purification par chromatographie sur gel de silice l'éluant étant un mélange toluène/acétate d'éthyle (9/1,v/v), on obtient le produit attendu sous forme de cristaux blancs avec un rendement de 74 %.
F = 96-99°C

**Exemple 16**

**2,4-dichloro-N-méthyl-N-(7-aminoheptyl)-3-[(2-méthylquinolin-8-yl) oxyméthyl]benzènesulfonamide, bis(trifluoroacétate)**

On prépare une solution de 2,5 g ($3,45.10^{-3}$ mole) du composé obtenu selon la préparation IX, dans 42 ml de dichlorométhane et on ajoute lentement 42 ml d'acide trifluoroacétique en maintenant une température d'environ 10°C. On laisse ensuite sous agitation pendant 45 min à température ambiante (20-25°C) puis on concentre sous pression réduite. Le résidu est repris avec du dichlorométhane, puis du toluène et concentré à sec. On obtient ainsi 2,57 g du produit attendu sous forme de cristaux beiges (Rendement = 98,8 %).
F = 66-68°C

**Exemple 17**

**2,4-dichloro-N-méthyl-N-(6-éthoxy-6-oxohexyl)-3-[(2-méthylquinolin-8-yl) oxyméthyl]benzènesulfonamide**

En opérant de façon analogue au procédé de l'exemple 4, au départ d'ester éthylique de l'acide 6-bromohexanoïque, on obtient, après purification par recristallisation dans l'alcool isopropylique, le produit attendu avec un rendement de 54 %.
F = 84-86°C

## Exemple 18

**2,4-dichloro-N-méthyl-N-(5-carboxypentyl)-3-[(2-méthylquinolin-8-yl)oxyméthyl]benzènesulfonamide**

On chauffe à 40-45°C pendant 3 heures une solution de 1,4 g (2,53.$10^{-3}$ mole) du composé obtenu selon l'exemple 17 dans 14 ml d'éthanol en présence de 5 ml d'une solution d'hydroxyde de sodium 1N. Le mélange réactionnel est ensuite concentré puis repris à l'eau et lavé avec de l'éther éthylique. La phase aqueuse est acidifiée par de l'acide chlorhydrique 1N jusqu'à pH 4 et extraite avec de l'acétate d'éthyle. La phase organique ainsi obtenue est lavée à l'eau, séchée sur sulfate de sodium et concentrée sous pression réduite. On obtient ainsi 1 g du produit attendu sous forme d'un solide blanc crème (Rendement = 76 %).
F = 98-102°C

## Exemple 19

**2,4-dichloro-N-méthyl-N-[6-(méthylamino)-6-oxohexyl]-3-[(2-méthylquinolin-8-yl)oxyméthyl]benzènesulfonamide**

En opérant de façon analogue au procédé de l'exemple 11, au départ de l'acide obtenu selon l'exemple 18, on obtient, après recristallisation dans le méthylcyclohexane, le produit attendu avec un rendement de 58 %.
F = 110-112°C

## PREPARATION X

**2,4-dichloro-N-méthyl-N-[8-[(tétrahydropyran-2-yl)oxy]octyl]-3-[(2-méthylquinolin-8-yl)oxyméthyl]benzènesulfonamide.**

En opérant de façon analogue au procédé de l'exemple 4, au départ de 2-(8-iodooctyl)tétrahydropyrane, et après purification par chromatographie sur gel de silice l'éluant étant un mélange cyclohexane/acétate d'éthyle (8/2, v/v), on obtient le produit attendu sous forme d'une huile jaune avec un rendement de 52 %.
RMN$^1$H ($CDCl_3$) : 1,20-1,90 (m,18H) ; 2,74 (s,3H) ; 3,08 (s,3H) ; 3,22 (t,2H) ; 3,37 (m,1H) ; 3,50 (m,1H) ; 3,72 (m,1H) ; 3,86 (m,1H) ; 4,56 (m,1H) ; 5,67 (s,2H) ; 7,23-7,50 (m,5H) ; 8,02 (d,1H) ; 8,07 (d,1H).

## PREPARATION XI

**2,4-dichloro-N-méthyl-N-[12-[(tétrahydropyran-2-yl)oxy]dodécyl]-3-[(2-méthylquinolin-8-yl)oxyméthyl]benzènesulfonamide**

En opérant de façon analogue au procédé de la préparation X, au départ de 2-(12-iodododecyl)tétrahydropyrane, on obtient le produit attendu sous forme d'une huile jaune.
RMN$^1$H ($CDCl_3$) : 1,10-1,90 (m,26H) ; 2,74 (s,3H) ; 2,86 (s,3H) ; 3,22 (t,2H) ; 3,37 (m,1H) ; 3,48 (m,1H) ; 3,75 (m,1H) ; 3,88 (m,1H) ; 4,57 (m,1H) ; 5,67 (s,2H) ; 7,23-7,50 (m,5H) ; 8,03 (d,1H) ; 8,08 (d,1H).

## Exemple 20

**2,4-dichloro-N-méthyl-N-(8-hydroxyoctyl)-3-[(2-méthylquinolin-8-yl)oxyméthyl]benzènesulfonamide**

On prépare une solution de 0,365 g (0,58.$10^{-3}$ mole) du composé obtenu selon la préparation X dans 4 ml de méthanol et on ajoute 89 mg (0,47.$10^{-3}$ mole) d'acide p-toluènesulfonique. Le mélange réactionnel est agité pendant 2 heures à température ambiante puis le méthanol est chassé sous pression réduite. Le résidu est repris à l'eau, extrait au dichlorométhane et les phases organiques rassemblées sont lavées à l'eau, séchées sur sulfate de sodium et concentrées sous pression réduite. Après purification par chromatographie sur gel de silice l'éluant étant le mélange cyclohexane/acétate d'éthyle (6/4, v/v), on obtient 250 mg du produit attendu sous forme de solide blanc (Rendement = 66 %).
F = 50°C

## Exemple 21

**2,4-dichloro-N-méthyl-N-(12-hydroxydodécyl)-3-[(2-méthylquinolin-8-yl)oxyméthyl]benzènesulfonamide**

En opérant de façon analogue au procédé de l'exemple 20, au départ du composé obtenu selon la préparation XI, on obtient le produit attendu sous forme d'un solide blanc pulvérulent.
F = 155°C

## PREPARATION XII

**N-[4-[bis-(1,1-diméthyléthoxycarbonyl)amino]butyl]-N-(1,1-diméthyléthyl)-2,4-dichloro-3-[(2-méthylquinolin-8-yl)oxyméthyl]benzènesulfonamide**

On prépare une solution de 1,55 g ($3{,}42.10^{-3}$ mole) du composé obtenu selon la préparation III, dans 45 ml d'acétonitrile et on ajoute 1,41 g ($10{,}2.10^{-3}$ mole) de carbonate de potassium et 1,50 g ($3{,}76.10^{-3}$ mole) d'acide (4-iodobutyl)(1,1-diméthyléthoxycarbonyl)carbamique, 1,1-diméthyléthyl ester. Le mélange réactionnel est chauffé à reflux du solvant sous agitation pendant 25 heures. Après refroidissement et filtration des sels minéraux, le filtrat est concentré à sec. Le résidu est repris à l'eau et extrait avec du dichlorométhane. La phase organique est lavée à l'eau jusqu'à neutralité, séchée sur sulfate de sodium et concentrée sous pression réduite. Le produit brut est purifié par chromatographie sur gel de silice l'éluant étant un mélange cyclohexane/éther éthylique (3/2, v/v). On obtient ainsi 0,635 g du produit attendu sous forme d'un solide blanc (Rendement =
25,6 %). F = 172-175° C

## Exemple 22

**N-(4-aminobutyl)-N-(1,1-diméthyléthyl)-2,4-dichloro-3-[(2-méthylquinolin-8-yl)oxyméthyl]benzènesulfonamide, dichlorohydrate**

On prépare une suspension de 1,21 g ($1{,}67.10^{-3}$ mole) du composé obtenu selon la préparation XII, dans 8,35 ml ($8{,}35.10^{-3}$ mole) d'une solution 1N de chlorure d'hydrogène dans l'acétate d'éthyle et on agite le mélange pendant 15 min à température ambiante. Le précipité résiduel est filtré et lavé sur filtre par de l'éther éthylique. On obtient ainsi 6,58 g du produit attendu sous forme d'un solide blanc (Rendement = 58 %).
F = 172-174°C

## Exemple 23

**N-(4-aminobutyl)-N-(1,1-diméthyléthyl)-2,4-dichloro-3-[(2-méthylquinolin-8-yl)oxyméthyl]benzènesulfonamide**

On ajoute 5 ml d'ammoniaque (solution à 30 %) à une solution de 0,563 g ($0{,}94.10^{-3}$ mole) du composé obtenu selon l'exemple 22, dans 70 ml d'eau et on agite le mélange pendant 30 min à température ambiante. Après extraction du milieu réactionnel à l'éther éthylique, la phase organique obtenue est lavée à l'eau, séchée sur sulfate de sodium et concentrée sous pression réduite. On obtient ainsi 0,20 g du produit attendu sous forme de cristaux beiges (Rendement = 40,4 %).
F = 142-144°C

## Exemple 24

**N-[4-[(1-oxoéthyl)amino]butyl]-N-(1,1-diméthyléthyl)-2,4-dichloro-3-[(2-méthylquinolin-8-yl)oxyméthyl]benzènesulfonamide**

A une solution de 0,120 g ($0{,}229.10^{-3}$ mole) du composé obtenu selon l'exemple 23 dans 1 ml d'acide acétique, on ajoute 0,109 ml ($1{,}145.10^{-3}$ mole) d'anhydride acétique et on maintient sous agitation pendant 24 heures à température ambiante. Le milieu réactionnel est ensuite concentré sous pression réduite et le résidu est repris en suspension par de l'éther éthylique. Le précipité obtenu est filtré et séché. On isole ainsi 0,272 g du produit attendu sous forme d'un solide blanc (Rendement = 21%).
F = 100-102°C (décomposition)

**Exemple 25**

**2,4-dichloro-N-(phénylméthyl)-N-(7-hydroxy-heptyl)-3-[(2-méthylquinolin-8-yl]oxyméthyl]benzènesulfonamide**

On prépare une solution de 0,5 g (0,83.$10^{-3}$ mole) du composé obtenu selon l'exemple 6 dans 10 ml de diméthylformamide et on ajoute 61,5 mg (2.$10^{-3}$ mole) d'hydrure de sodium à 80 % dans l'huile. Après 30 min d'agitation du mélange à température ambiante, on ajoute 220 mg (1,12.$10^{-3}$ mole) de 7-bromoheptanol. Le mélange réactionnel est agité pendant 15 heures à 40°C, puis refroidi, hydrolysé sur de l'eau et extrait à l'acétate d'éthyle. Les phases organiques rassemblées sont lavées à l'eau jusqu'à neutralité, séchées sur sulfate de sodium et concentrées sous pression réduite. Après purification du résidu par chromatographie sur gel de silice l'éluant étant un mélange cyclohexane/acétate d'éthyle (7/3 ; v/v), on obtient 150 mg du produit attendu sous forme d'un solide blanc (Rendement = 24 %).
RMN$^1$H ($CDCl_3$) : 0,90-1,60 (m,10H) ; 2,75 (s,3H) ; 3,20 (m,2H) ; 3,56 (m,2H) ; 4,53 (m,2H) ; 5,68 (m,2H) ; 7,26-7,47 (m,10H) ; 8,03 (d,1H) ; 8,09 (d,1H).

**Exemple 26**

**2,4-dichloro-N-(phénylméthyl)-N-(8-éthoxy-8-oxooctyl)-3-[(2-méthylquinolin-8-yl)oxyméthyl]benzènesulfonamide**

En opérant de façon analogue au procédé de l'exemple 4, au départ du composé obtenu selon l'exemple 6 et de 8-bromooctanoate d'éthyle, on obtient, après purification par recristallisation dans l'alcool isopropylique, le produit attendu avec un rendement de 37 %.
F = 117°C

**Exemple 27**

**2,4-dichloro-N-(phénylméthyl)-N-(7-carboxyheptyl)-3-[(2-méthylquinolin-8-yl)oxyméthyl]benzènesulfonamide**

On prépare une suspension de 1,1 g (1,67.$10^{-3}$ mole) du composé obtenu selon l'exemple 26, dans 100 ml d'éthanol et on ajoute une solution de 0,1 g (2,5.$10^{-3}$ mole) d'hydroxyde de sodium dans 10 ml d'eau. Le mélange réactionnel est chauffé à reflux pendant 3 heures, puis le solvant est évaporé sous pression réduite et le résidu est repris à l'eau. La phase aqueuse ainsi obtenue est acidifiée à pH 5 à l'aide d'acide chlorhydrique 1N. Le précipité formé est filtré, lavé à l'eau, séché sous vide à 60°C, puis purifié par chromatographie sur gel de silice l'éluant étant un mélange dichlorométhane/méthanol (9/1, v/v). Après recristallisation dans le mélange toluène/éther isopropylique, on obtient 0,8 g du produit attendu sous forme d'un solide blanc crème (Rendement = 76 %).
F = 100°C

**Exemple 28**

**2,4-dichloro-N-(phénylméthyl)-N-[8-(méthylamino)-8-oxooctyl]-3-[(2-méthylquinolin-8-yl)oxyméthyl]benzènesulfonamide**

En opérant de façon analogue au procédé de l'exemple 10, au départ de l'acide obtenu selon l'exemple 27 et d'une solution de méthylamine dans l'éthanol, on obtient, après purification par chromatographie sur gel de silice l'éluant étant un mélange dichlorométhane/méthanol (98/2, v/v) puis cristallisation dans l'éther isopropylique, le produit attendu avec un rendement de 98 %.
F = 120°C

L'activité de certains des produits selon l'invention a été évaluée en fonction de leur aptitude à se lier aux récepteurs de la bradykinine. En effet, les kinines, dont le principal représentant est la bradykinine, forment un groupe de petits peptides qui contribuent de façon importante à la réponse inflammatoire et apparaissent de ce fait impliqués dans la pathophysiologie des maladies inflammatoires. De plus, la bradykinine est un des agents algésiants parmi les plus puissants connus. Les kinines activent deux types de récepteurs appelés respectivement $B_1$ et $B_2$. Le récepteur $B_2$ appartient à la grande famille des récepteurs à sept domaines transmembranaires couplés aux G-protéines. Nous décrivons dans la présente invention des composés se liant au récepteur $B_2$ et bloquant de ce fait la fixation de la bradykinine.

Le test pharmacologique que nous avons utilisé est le suivant : des segments d'iléon de cobayes mâles de

souche Dunkin-Hartley (Iffa Credo, l'Arbresle, France) sont isolés et broyés dans le tampon TES suivant : TES 25mM, 1,10-phénanthroline 1mM (pH 6.8), bacitracine 140 µg/ml, BSA 1g/l. Les membranes sont ensuite isolées par centrifugation (18000 tours par minute ; 20 min ; 4° C). Les études de liaison sont effectuées dans le tampon TES en utilisant la [$^3$H]-bradykinine (120 pM), 50 µg de protéine membranaire par essai (volume final 500 µl) avec un temps d'équilibre de 90 min à 20°C. On détermine ensuite le taux (en pourcentage) d'inhibition de la fixation de [$^3$H]-bradykinine en présence de l'un des composés selon l'invention à tester à une concentration de $10^{-6}$ M.

Les résultats obtenus (notés "activité") lors de ces essais sont consignés dans le tableau I ci-après en regard des exemples figurant dans la description.

Les composés de la présente invention qui inhibent la liaison de la [$^3$H] bradykinine au récepteur $B_2$ de cobaye (voir tableau I) se lient également au récepteur $B_2$ humain cloné et transfecté de façon stable dans des cellules CHO (Chinese Hamster Ovary Cells). Ainsi dans ce test, certains composés inhibent à la concentration de 10 µM d'au moins 95 % la fixation de la [$^3$H]-bradykinine au récepteur $B_2$.

Les composés de la présente invention peuvent être utiles dans le traitement de nombreuses pathologies impliquant la bradykinine ou ses homologues. Parmi ces pathologies, on inclut les chocs septiques et hémorragiques, les réactions anaphylactiques, l'arthrose, la polyarthrite rhumatoïde, les rhinites, l'asthme, les maladies inflammatoires du tractus gastrointestinal (par ex. colites, rectites, maladie de Crohn), la pancréatite, certains carcinomes, l'angiooedème héréditaire, la migraine, les accidents vasculaires cérébraux, certains désordres neurologiques, les états inflammatoires vasculaires (par exemple : athérosclérose et artérite des membres inférieurs), les états douloureux (par exemple douleur dentaire, douleurs menstruelles), les contractions utérines prématurées, la cystite et les brûlures.

Les composés de la présente invention, qui peuvent être utilisés sous forme de base libre ou de leurs sels d'addition non-toxiques, en association avec un excipient physiologiquement acceptable, seront en général prescrits en thérapie à des doses d'environ 1 à 1000 mg/jour, sous une forme administrable par voie orale, par injection intraveineuse, intramusculaire ou sous-cutanée, par voie transdermique, par le moyen d'aérosols ou par le moyen de suppositoires.

Les composés pourront également être administrés par voie topique, par exemple sous forme de gels ou de pommades.

Les composés de la présente invention trouvent également leur utilité, en tant que réactifs pharmacologiques, notamment pour l'étude des interactions hormone-récepteur. L'utilisation en tant que réactif pharmacologique peut faire appel à un dérivé radiomarqué de l'un des composés selon l'invention (par exemple avec du tritium [$^3$H] ou de soufre [$^{35}$S]), dans le but d'obtenir un radio-ligand destiné à des études conformationnelles du récepteur $B_2$ de la bradykinine, ou des tests de fixation à ce type de récepteur, par exemple pour l'évaluation de nouveaux composés susceptibles de présenter une affinité pour le récepteur $B_2$ de la bradykinine.

# Tableau I

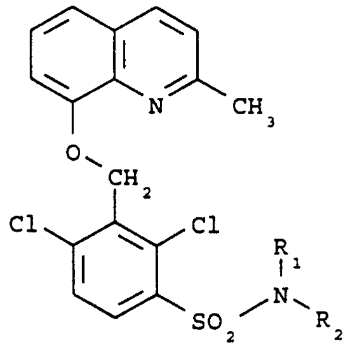

| Exemple n° | R1 | R2 | Activité % |
|---|---|---|---|
| 1 | $-CH_3$ | $-C(CH_3)_3$ | - |
| 2 | $-CH_3$ | -H | - |
| 3 | -H | -H | - |
| 4 | $-CH_3$ | 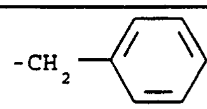 | - |
| 5 | $-C(CH_3)_3$ | 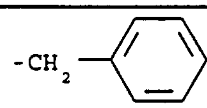 | - |
| 6 | -H | 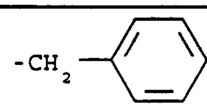 | - |
| 7 | $-CH_3$ | 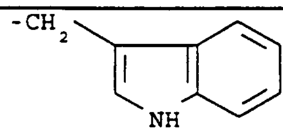 | 97,6 |
| 8* | $-CH_3$ | 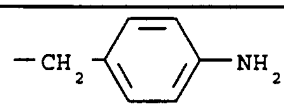 | 99,5 |
| 9 | $-CH_3$ | 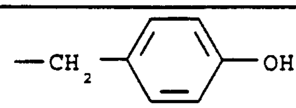 | 99,5 |
| 10 | $-CH_3$ | 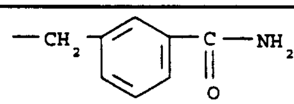 | 99,8 |
| 11 | $-CH_3$ | 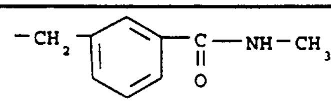 | 100 |
| 12 | $-CH_3$ | 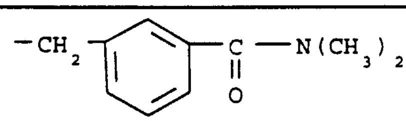 | 96,9 |

| | | | |
|---|---|---|---|
| 13 | -CH3 | 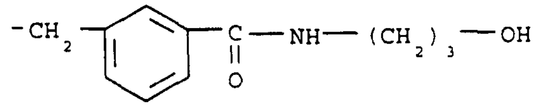 | 99,3 |
| 14 | -CH3 | 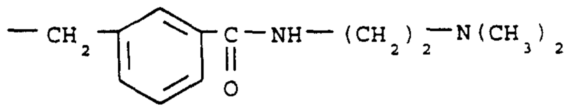 | 100 |
| 15 | -CH3 | 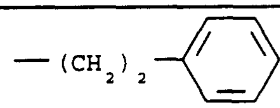 | 97,9 |
| 16** | -CH3 | $-(CH_2)_7-NH_2$ | 100 |
| 17 | -CH3 | $-(CH_2)_5-COOC_2H_5$ | - |
| 18 | -CH3 | $-(CH_2)_5-COOH$ | - |
| 19 | -CH3 | $-(CH_2)_5-CONHCH_3$ | 99,0 |
| 20 | -CH3 | $-(CH_2)_8-OH$ | 95,7 |
| 21 | -CH3 | $-(CH_2)_{12}-OH$ | 94,3 |
| 22* | -C(CH3)3 | $-(CH_2)_4-NH_2$ | - |
| 23 | -C(CH3)3 | $-(CH_2)_4-NH_2$ | 92,8 |
| 24 | -C(CH3)3 | $-(CH_2)_4-NH-CO-CH_3$ | 90,5 |
| 25 | 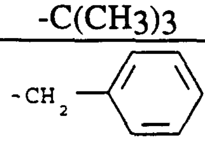 | $-(CH_2)_7-OH$ | 95,1 |
| 26 | 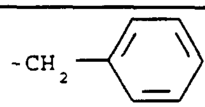 | $-(CH_2)_7-COOC_2H_5$ | - |
| 27 | 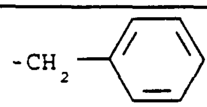 | $-(CH_2)_7-COOH$ | - |
| 28 | 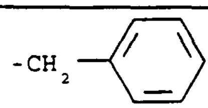 | $-(CH_2)_7-CONHCH_3$ | 98,0 |
| 29 | -H | -C(CH3)3 | - |

Notes : * : dichlorhydrate, ** : bis(trifluoroacétate)

**Revendications**

**1.** Composé de benzènesulfonamide, **caractérisé en ce qu'**il est choisi parmi l'ensemble constitué par :

(i) les composés de formule :

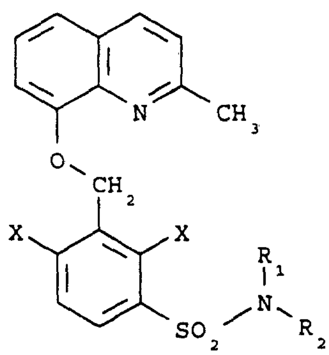

(I)

dans laquelle :

X représente un atome d'halogène,
$R_1$ et $R_2$, identiques ou différents, représentent chacun un atome d'hydrogène ou un groupe $-A-B-R_3$,
A représente une chaîne alkylène en $C_1$-$C_{12}$, linéaire ou ramifiée,
B représente

une simple liaison,
un groupe phénylène de structure

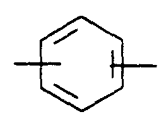

par rapport auquel les substituants A et $R_3$ sont en position ortho, méta ou para, ou
un groupe indolyle divalent de structure

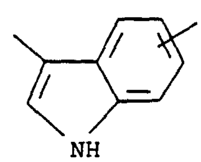

$R_3$ représente -H,-OH,$-NR_4R_5$ ou $-COR_6$,
$R_6$ représente un groupe -OH, $-OCH_3$, $-OC_2H_5$ ou $-NR_4R_5$,
$R_4$ et $R_5$, identiques ou différents représentent chacun un atome d'hydrogène, un groupe alkyle en $C_1$-$C_4$ à chaîne hydrocarbonée linéaire ou ramifiée, un groupe $-(CH_2)_n-OH$, un groupe $-(CH_2)_n-$ $N(CH_3)_2$ ou un groupe $-CO-CH_3$,
n représente un nombre entier ayant pour valeur 2, 3 ou 4 ; et,

(ii) leurs sels d'addition.

**2.** Composé selon la revendication 1, **caractérisé en ce qu'**il répond à la formule :

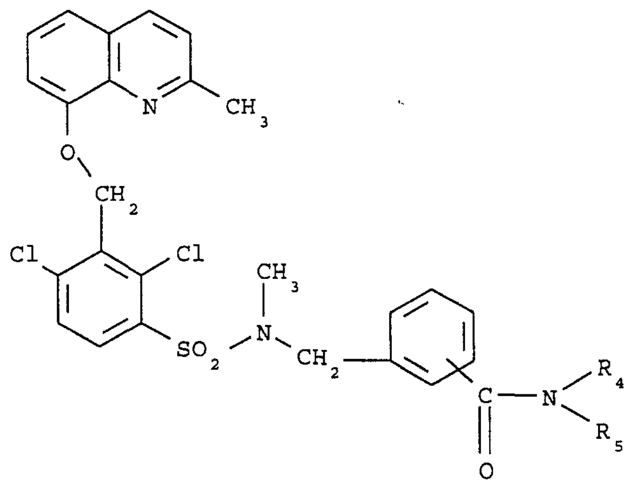

dans laquelle le groupe amide peut être en position ortho, méta ou para et, $R_4$ et $R_5$, identiques ou différents, représentent chacun :

un atome d'hydrogène,
un groupe alkyle en $C_1$-$C_4$, linéaire ou ramifié,
un groupe $-(CH_2)_n-OH$, ou
un groupe $-(CH_2)_n-N(CH_3)_2$,
où n représente un nombre entier valant 2, 3 ou 4 ; et,

ses sels d'addition d'acide non-toxiques.

3. Utilisation d'une substance choisie parmi l'ensemble constitué par les composés de formule I selon la revendication 1 et leurs sels d'addition non toxiques pour l'obtention d'un médicament destiné à une utilisation en thérapeutique vis à vis d'états pathologiques impliquant la bradykinine ou ses analogues.

4. Utilisation selon la revendication 3, **caractérisée en ce que** ladite substance intervient dans l'obtention d'un médicament destiné à une utilisation en thérapeutique pour le traitement des algies.

5. Utilisation selon la revendication 3, **caractérisée en ce que** ladite substance intervient dans l'obtention d'un médicament destiné à une utilisation en thérapeutique pour le traitement d'états inflammatoires.

6. Composition thérapeutique **caractérisée en ce qu'**elle renferme, en association avec un excipient physiologiquement acceptable, au moins un composé choisi parmi l'ensemble constitué par les composés de formule I selon la revendication 1 et leurs sels d'addition non-toxiques.

7. Utilisation d'un composé selon la revendication 1 en tant que réactif pour l'étude de la conformation du récepteur $B_2$ de la bradykinine ou pour l'étude des composés susceptibles de présenter une affinité pour ledit récepteur.

8. Procédé pour la préparation d'un composé de benzènesulfonamide de formule I selon la revendication 1 et de ses sels d'addition, ledit procédé étant **caractérisé en ce qu'**il comprend la réaction de condensation de la 8-hydroxy-2-méthylquinoléine sous la forme phénate de formule :

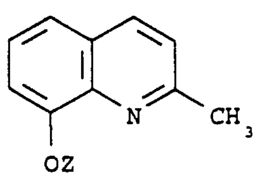

(VIII)

où Z est un atome de métal alcalin,
avec un toluènesulfonamide halogéné de formule :

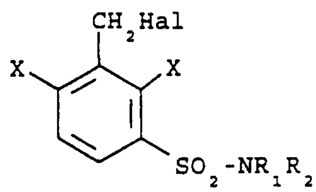

(IX)

dans laquelle Hal représente un atome d'halogène, et X, $R_1$ et $R_2$ sont définis comme indiqué ci-dessus, les fonctions susceptibles d'être contenues dans $R_1$ et/ou $R_2$ et d'intervenir dans la réaction VIII + IX → I étant protégées.

**9.** Procédé selon la revendication 8, **caractérisé en ce qu'**il comprend le schéma réactionnel suivant :

(α) on synthétise, selon la réaction VIII + IX → I , un composé de formule :

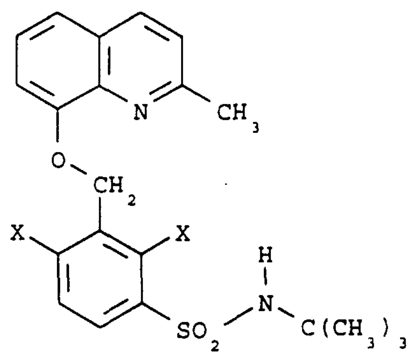

(V)

(β) si nécessaire, on traite le composé de formule V pour remplacer le groupe $R_2$ = H par un groupe $R_2$ différent de H et obtenir un composé de formule :

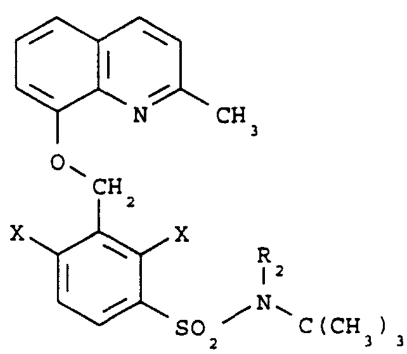

(Va)

(γ) si nécessaire, on traite le composé de formule Va, ainsi obtenu, pour remplacer le groupe $R_1 = C(CH_3)_3$ par l'atome d'hydrogène et obtenir un composé de formule :

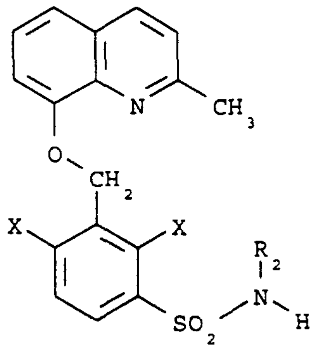

(Vb)

puis,

(δ) si nécessaire, on traite le composé de formule Vb, ainsi obtenu, pour remplacer le groupe $R_1$ = H par un groupe $R_1$ différent de H et obtenir un composé de formule I où $R_1$ et $R_2$ sont chacun différents de H,

et,

(ε) si nécessaire, on obtient le sel d'addition d'acide de la base libre résultant du stade (β), (γ) ou (δ) en faisant réagir ladite base libre avec un acide.

10. Composé intermédiaire, utile pour la préparation des composés selon la revendication 1, **caractérisé en ce qu'**il répond à la formule :

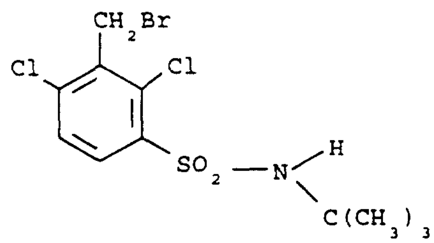

**Patentansprüche**

1. Benzolsulfonamid-Verbindung, **dadurch gekennzeichnet, dass** sie aus der Gesamtheit ausgewählt ist, die von

a) Verbindungen mit der Formel

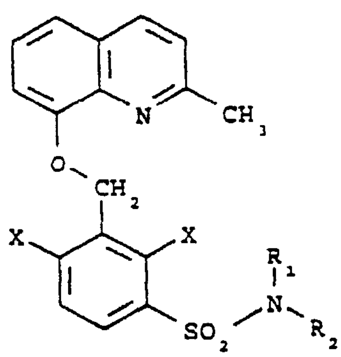

(I)

in welcher

X ein Halogenatom bedeutet,
$R_1$ und $R_2$, die gegebenenfalls verschieden sind, jeweils ein Wasserstoffatom oder eine -A-B-R3-Gruppe bedeuten,
A eine geradkettige oder verzweigte $C_1$- bis $C_{12}$-Alkylenkette,
B eine Einfachbindung,

eine Phenylengruppe mit der Struktur

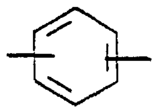

in Bezug auf welche die Substituenten A und $R_3$ sich in ortho-, meta- oder para-Stellung befinden, oder eine zweiwertige Indolylgruppe mit der Struktur

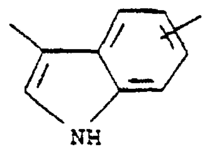

$R_3$ -H, -OH, $-NR_4R_5$ oder $-COR_6$ und
$R_6$ eine -OH-, $-OCH_3$-, $-OC_2H_5$- oder $-NR_4R_5$-Gruppe bedeutet und
$R_4$ und $R_5$, die gegebenenfalls verschieden sind, jeweils ein Wasserstoffatom, eine $C_1$- bis $C_4$-Alkylgruppe mit geradkettiger oder verzweigter Kohlenwasserstoffkette und eine $-(CH_2)_n-OH$-, $-(CH_2)_n-N(CH_3)_2$- oder $-CO-CH_3$-Gruppe bedeuten, wobei n ganzzahlig mit 2, 3 oder 4 ist, und

b) ihren Additionssalzen gebildet wird.

**2.** Verbindung nach Anspruch 1, **dadurch gekennzeichnet, dass** sie der Formel

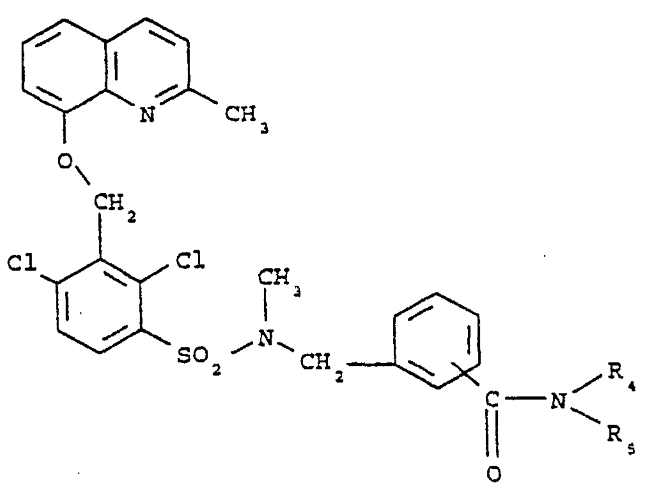

in welcher die Amidgruppe sich in ortho-, meta- oder para-Stellung befinden kann und $R_4$ und $R_5$, die gegebenenfalls verschieden sind, jeweils

ein Wasserstoffatom,
eine geradkettige oder verzweigte $C_1$- bis $C_4$-Alkylgruppe,
eine $-(CH_2)_n$-OH-Gruppe oder
eine-$(CH_2)_n$-$N(CH_3)_2$-Gruppe

bedeuten, wobei n ganzzahlig mit 2, 3 oder 4 ist,
und ihren nichttoxischen Säureadditionssalzen entspricht.

**3.** Verwendung einer Substanz, die aus der Gesamtheit ausgewählt ist, die von den Verbindungen mit der Formel I nach Anspruch 1 und deren nichttoxischen Additionssalzen gebildet wird, zur Herstellung eines Arzneimittels, das für eine therapeutische Anwendung bei Erkrankungen vorgesehen ist, an denen Bradykinin oder dessen Analogen beteiligt sind.

**4.** Verwendung nach Anspruch 3, **dadurch gekennzeichnet, dass** die Substanz an der Herstellung eines Arzneimittels beteiligt ist, das für eine therapeutische Anwendung bei der Behandlung von Schmerzzuständen vorgesehen ist.

**5.** Verwendung nach Anspruch 3, **dadurch gekennzeichnet, dass** die Substanz an der Herstellung eines Arzneimittels beteiligt ist, das für eine therapeutische Anwendung bei der Behandlung von Entzündungszuständen vorgesehen ist.

**6.** Therapeutische Zusammensetzung, **dadurch gekennzeichnet, dass** sie zusammen mit einem physiologisch verträglichen Arzneimittelträger mindestens eine Verbindung enthält, welche aus der Gesamtheit ausgewählt ist, die von den Verbindungen mit der Formel I nach Anspruch 1 und deren nichttoxischen Additionssalzen gebildet wird.

**7.** Verwendung einer Verbindung nach Anspruch 1 als Reagenz zur Untersuchung der Konformation des $B_2$-Rezeptors für das Bradykinin oder zur Untersuchung der Verbindungen, die in der Lage sind, eine Affinität gegenüber diesem Rezeptor aufzuweisen.

**8.** Verfahren zur Herstellung einer Benzolsulfonamid-Verbindung mit der Formel I nach Anspruch 1 und ihren Additionssalzen, wobei das Verfahren **dadurch gekennzeichnet** ist, dass es die Kondensationsreaktion des 8-Hydroxy-2-methylchinolins in Phenatform mit der Formel

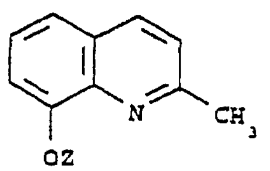

(VIII)

worin Z ein Alkalimetallatom bedeutet, mit halogeniertem Toluolsulfonamid mit der Formel

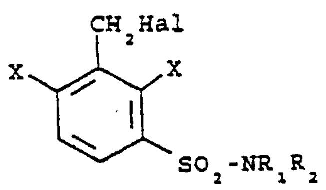

(IX)

umfasst, in welcher Hal ein Halogenatom bedeutet und X, $R_1$ und $R_2$ wie zuvor definiert sind, wobei die Funktionen, die in der Lage sind, in $R_1$ und/oder $R_2$ enthalten zu sein und an der Reaktion VIII + IX $\rightarrow$ I beteiligt sein zu können, geschützt sind.

**9.** Verfahren nach Anspruch 8, **dadurch gekennzeichnet, dass** es folgendes Reaktionsschema umfasst:

(α) Synthese gemäß der Reaktion VIII + IX → I einer Verbindung mit der Formel

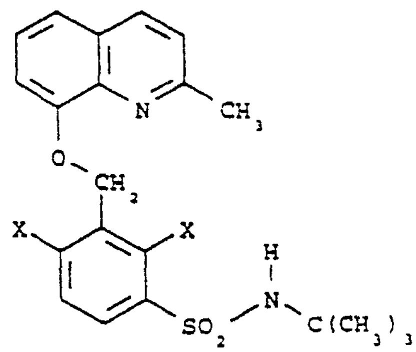

(V)

(β) erforderlichenfalls Behandlung der Verbindung mit der Formel V, um die Gruppe $R_2$ = H durch eine von H verschiedene Gruppe $R_2$ zu ersetzen und eine Verbindung mit der Formel

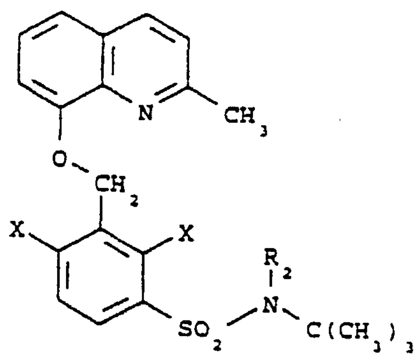

(Va)

zu erhalten,

(γ) erforderlichenfalls Behandlung der so erhaltenen Verbindung mit der Formel Va, um die Gruppe $R_1$ = C $(CH_3)_3$ durch ein Wasserstoffatom zu ersetzen und eine Verbindung mit der Formel

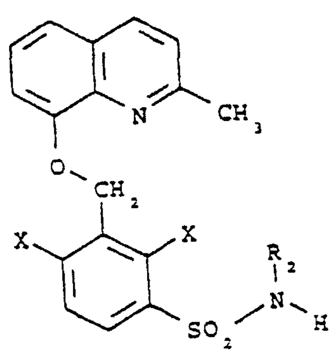

(Vb)

zu erhalten, anschließend

(δ) erforderlichenfalls Behandlung der so erhaltenen Verbindung mit der Formel Vb, um die Gruppe $R_1$ = H durch eine von H verschiedene Gruppe $R_1$ zu ersetzen und eine Verbindung mit der Formel I zu erhalten, worin $R_1$ und $R_2$ jeweils von H verschieden sind, und

(ε) erforderlichenfalls Erhalten des Säureadditionssalzes der freien Base, die aus der Stufe (β), (γ) oder (δ) resultiert, indem diese freie Base mit einer Säure umgesetzt wird.

**10.** Zwischenverbindung, die für die Herstellung der Verbindungen nach Anspruch 1 nützlich ist, **dadurch gekennzeichnet, dass** sie der Formel entspricht:

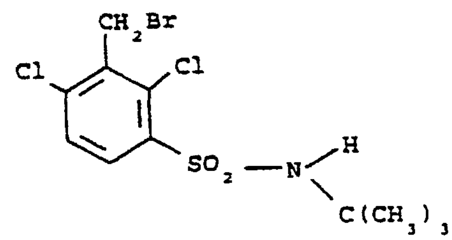

**Claims**

**1.** A benzenesulfonamide compound, **characterized in that** it is selected from the group consisting of:

(i) the compounds of the formula

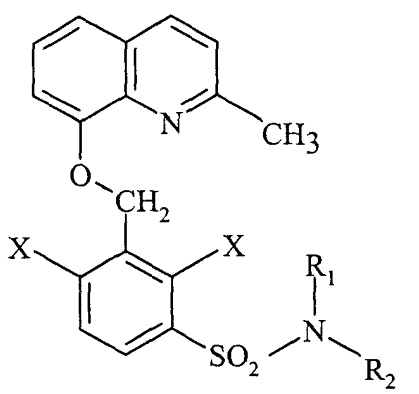

(I)

in which:

X is a halogen atom,
$R_1$ and $R_2$, which are identical or different, are each a hydrogen atom or a group -A-B-$R_3$,
A is a linear or branched $C_1$-$C_{12}$-alkylene chain,
B is

a single bond,
a phenylene group of the structure

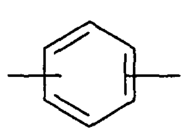

relative to which the substituents A and $R_3$ are in the ortho, meta or para position, or a divalent indolyl group of the structure

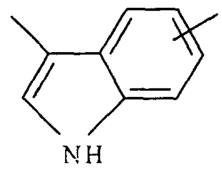

$R_3$ is -H, -OH, $-NR_4R_5$ or $-COR_6$,
$R_6$ is a group -OH, $-OCH_3$, $-OC_2H_5$ or $-NR_4R_5$,
$R_4$ and $R_5$, which are identical or different, are each a hydrogen atom, a $C_1$-$C_4$-alkyl group with a linear or branched hydrocarbon chain, a group $-(CH_2)_n-OH$, a group $-(CH_2)_n-N(CH_3)_2$ or a group $-CO-CH_3$, and n is an integer with a value of 2, 3 or 4; and

(ii) their addition salts.

**2.** A compound according to claim 1, **characterized in that** it has the formula

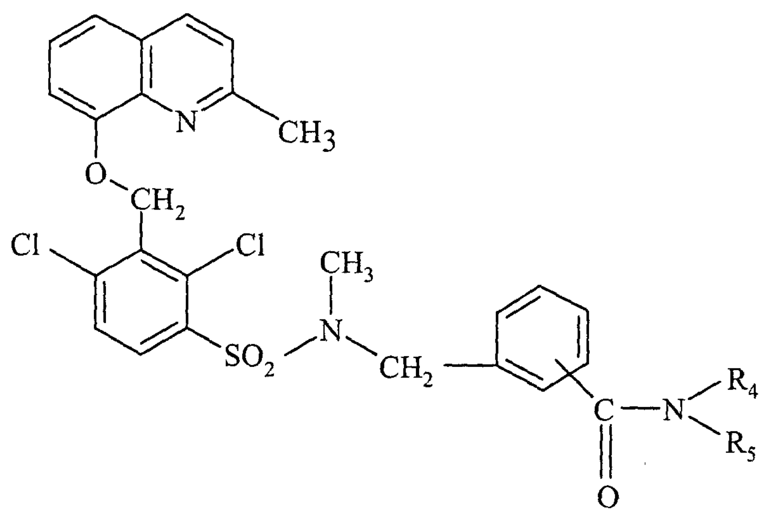

in which the amide group can be in the ortho, meta or para position and $R_4$ and $R_5$, which are identical or different, are each:

a hydrogen atom,
a linear or branched $C_1$-$C_4$-alkyl group,
a group $-(CH_2)_n-OH$, or
a group $-(CH_2)_n-N(CH_3)_2$,
where n is an integer with a value of 2, 3 or 4; and
its non-toxic acid addition salts.

**3.** Use of a substance selected from the group consisting of the compounds of formula I according to claim 1 and their non-toxic addition salts for obtaining a drug intended for use in therapeutics to combat pathological conditions involving bradykinin or its analogs.

**4.** Use according to claim 3, **characterized in that** said substance is employed in obtaining a drug intended for use

in therapeutics for the treatment of pain.

**5.** Use according to claim 3, **characterized in that** said substance is employed in obtaining a drug intended for use in therapeutics for the treatment of inflammatory states.

**6.** A therapeutics composition, **characterized in that** it contains, in association with a physiologically acceptable excipient, at least one compound selected from the group consisting of the compounds of formula I according to claim 1 and their non-toxic addition salts.

**7.** Use of a compound according to claim 1 as a pharmacological reagent, for the study of the conformation of the bradykinin $B_2$ receptor or the study of compounds capable of showing an affinity for said receptor.

**8.** A process for the preparation of a benzenesulfonamide compound of formula I according to claim 1 and its addition salts, said process being **characterized in that** it comprises a condensation reaction of 8-hydroxy-2-methylquinoline in the form of a phenate of the formula

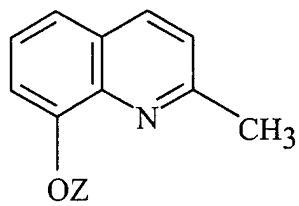

(VIII)

in which Z is an alkali metal atom,
with a halogenated toluenesulfonamide of the formula

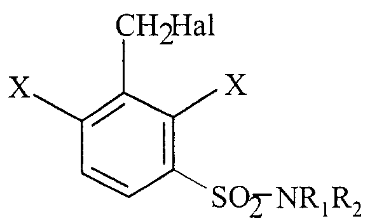

(IX)

in which Hal is a halogen atom and X, $R_1$ and $R_2$ are defined as indicated above, the functional groups which may be contained in $R_1$ and/or $R_2$ and may take part in the reaction VIII + IX → I being protected.

**9.** A process according to claim 8, **characterized in that** it comprises the following reaction scheme:

(α) a compound of the formula

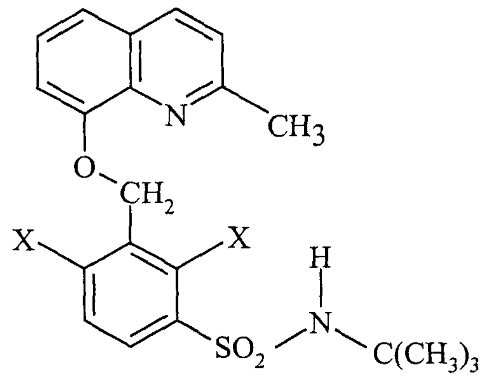

(V)

is synthesized according to the reaction VIII + IX → I,

(β) if necessary, the compound of formula V is treated in order to replace the group $R_2$ = H with a group $R_2$ other than H to give a compound of the formula

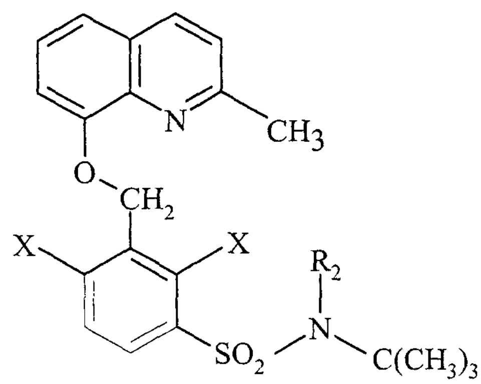

(Va)

(γ) if necessary, the resulting compound of formula Va is treated in order to replace the group $R_1 = C(CH_3)_3$ with the hydrogen atom to give a compound of the formula

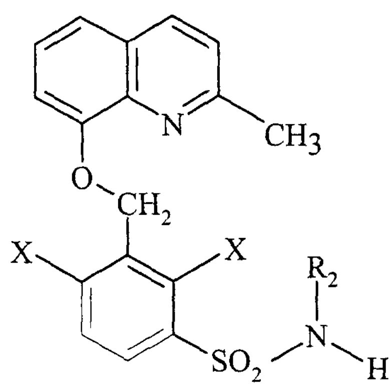

(Vb)

then

(δ) if necessary, the resulting compound of formula Vb is treated in order to replace the group $R_1$ = H with a group $R_1$ other than H to give a compound of formula I in which both $R_1$ and $R_2$ are other than H,

and

(ε) if necessary, the acid addition salt of the free base resulting from stage (β), (γ) or (δ) is obtained by reacting said free base with an acid.

**10.** An intermediate useful for the preparation of the compounds according to claim 1, **characterized in that** it has the formula